# EUROPÄISCHE PATENTSCHRIFT

(11) **EP 1 893 563 B1**
(45) Veröffentlichungstag und Bekanntmachung des Hinweises auf die Patenterteilung: **08.06.2011**
(21) Anmeldenummer: 06743137.9
(22) Anmeldetag: 09.06.2006
(51) Int. Cl.: C07C 263/04, C07C 269/00, C07C 269/04, C07C 273/18, C07C 265/12, C07C 271/28, C07C 275/28

(54) **HERSTELLUNG VON N-ARYLCARBAMATEN UND N-ARYLISOCYANATEN**
PRODUCTION OF N-ARYL CARBAMATES AND N-ARYL ISOCYANATES
FABRICATION DE N-ARYLCARBAMATES ET DE N-ARYLISOCYANATES

(30) Priorität: 09.06.2005 DE 102005026500
(43) Veröffentlichungstag der Anmeldung: 05.03.2008
(73) Patentinhaber: PHILIPPS-UNIVERSITÄT MARBURG, D-35032 Marburg (DE)
(72) Erfinder: SUNDERMEYER, Jörg, 35041 Marburg (DE); MEI, Fuming, Wuhan, Hubei 430074 (CN)
(86) Internationale Anmeldenummer: PCT/EP2006/005516
(87) Internationale Veröffentlichungsnummer: WO 2006/131381

(56) Entgegenhaltungen:
- US-A- 3 481 967
- M. M. TAQUI KHAN ET AL.: "Reductive carbonylation of nitrobenzene to phenylurethane catalyzed by Ru(III)-Schiff Base complexes" J MOL CATAL, Bd. 57, 1990, Seiten 301-305, XP009073364

## Beschreibung

Gegenstand der Erfindung ist ein Verfahren zur Herstellung von N-Arylcarbamaten (Urethanen) und/oder N-Arylisocyanaten durch reduktive Carbonylierung von aromatischen Nitroverbindungen in Gegenwart von Kohlenmonoxid und Alkoholen.

Aryl-diisocyanate, beispielsweise Toluoldiisocyanat TDI, sind technisch bedeutende Zwischenprodukte für die Gewinnung von Polyurethanen, die zum Beispiel als Schaumstoffe vielfache Anwendung finden. Sie werden gewöhnlich aus den Dinitroaromaten gewonnen, die zunächst zu cancerogenen aromatischen Diaminen (Anilinen) reduziert werden müssen. Deren Umsetzung mit hoch giftigem Phosgen liefert die bifunktionellen Isocyanate, wobei große Mengen von toxischem Chlorwasserstoff als Abfall anfallen.

Seitens der Industrie besteht deshalb großes Interesse an der Entwicklung von Syntheseverfahren, die ohne die Verwendung von Phosgen auskommen und den lästigen Abfallstoff Chlorwasserstoff vermeiden. Außerdem ist es ein Vorteil, wenn Nitroaromaten nicht erst zu aromatischen Aminoverbindungen reduziert, sondern direkt in N-Arylcarbamate und/oder N-Arylisocyanate umgewandelt werden können.

So ist aus der US-Patentschrift 3 481 967 bereits ein Verfahren zur Herstellung von aromatischen Isocyanaten bekannt, bei dem aromatische Nitroverbindungen und Kohlenmonoxid in Gegenwart von Übergangsmetallkatalysatoren hergestellt werden können. Diese Übergangsmetallkatalysatoren umfassen Kobaltiodid und Titantetrachlorid. Technisch brauchbare Verfahren müssen sich jedoch durch einen hohen Umsatz und eine Selektivität von möglichst über 90% auszeichnen, um mit Erfolg einsetzbar zu sein. Diese Anforderungen konnten bisher von Verfahren der reduktiven Carbonylierung, die von aromatischen Nitroverbindungen ausgehen, nicht erfüllt werden.

Deshalb hat sich die industrielle und wissenschaftliche Forschung ganz überwiegend auf die Entwicklung von Verfahren konzentriert, bei denen die zunächst aus aromatischen Nitroverbindungen hergestellten aromatischen Aminoverbindungen in Gegenwart von Kohlenmonoxid und einer organischen Hydroxylgruppen enthaltenden Verbindung durch oxidative Carbonylierung in N-Arylcarbamate und/oder N-Arylisocyanate umgewandelt werden. Hierzu ist der Zusatz eines Oxidationsmittels und die Gegenwart eines Katalysators erforderlich, wobei allerdings nahezu ausschließlich teure Edelmetallkatalysatoren eingesetzt werden konnten. Ein Beispiel hierfür ist das US-Patent 5 194 660, bei dem für die oxidative Carbonylierung von aromatischen Aminen in Gegenwart von Kohlenmonoxid und einer organischen Hydroxylgruppen enthaltenden Verbindung Sauerstoff als Oxidationsmittel und makrozyklische Edelmetallkomplexkatalysatoren eingesetzt werden. Zu den Nachteilen dieses Verfahrens ist der Einsatz großer Mengen an Lithiumiodid als Promotor und die technisch innerhalb der Explosionsgrenzen nicht sicher zu beherrschende Mischung der Reaktivgase CO und O₂ zu zählen.

Daher haben Verfahren der reduktiven Carbonylierung gegenüber der oxidativen Carbonylierung in Gegenwart von Sauerstoff Sicherheitsvorteile. Alle bisher bekannten Verfahren zur reduktiven Carbonylierung von Nitroverbindungen sind jedoch ebenfalls mit erheblichen Nachteilen behaftet, weil sie entweder nicht von den als Ausgangsstoffen besonders preiswerten Nitroaromaten ausgehen, insbesondere aber, weil sie teure und daher gegenüber dem klassischen Phosgenierungsverfahren unwirtschaftliche Edelmetallkatalysatoren verwenden, die häufig nicht einmal die Umsatz- und Selektivitätswerte von über 90% erreichen. Eine Übersicht über aktuelle Entwicklungen bieten F. Paul, Coordination Chemistry Reviews 203 (2000) 269-323 und F. Ragaini et al. Advanced Synthesis & Catalysis 346 (2004) 63-71.

Bekannt ist des Weiteren ein Verfahren zur reduktiven Carbonylierung von Nitrobenzol zur Herstellung von Phenylurethan, wobei als Katalysatoren Ruthenium-Schiffbasen-Komplexe zum Einsatz kommen, darunter auch Salen- bzw. Salophen-Komplexe (M.M. Taqui Khan et al.: "Reductive Carbonylation of Nitrobenzene to Phenylurethane Catalyzed by Ru(III)-Schiff Base Complexes"J Mol Catal, Bd.57, 1990, Seiten 301-305).

Es stellte sich deshalb die Aufgabe, ein Verfahren zur Herstellung von N-Arylcarbamaten und/oder N-Arylisocyanaten zu entwickeln, das ausgehend von aromatischen Nitroverbindungen durch reduktive Carbonylierung in hohen Ausbeuten und mit großer Selektivität angewendet werden kann.

Diese vielfältigen Anforderungen lassen sich nur durch den Einsatz spezifischer Katalysatoren lösen, wie sie Gegenstand dieser Erfindung sind.

Es wurde nun gefunden, dass ein Verfahren zur Herstellung von N-Arylcarbamaten (Urethanen) und/oder N-Arylisocyanaten durch reduktive Carbonylierung von aromatischen Nitroverbindungen in Gegenwart von Kohlenmonoxid und organischen Hydroxylgruppen tragenden Verbindungen zu ausgezeichneten Ergebnissen führt, wenn die Carbonylierung in Anwesenheit von Metall-Komplexkatalysatoren durchgeführt wird, die anionische N,O-Chelatliganden des allgemeinen Typs [M(N∼O)¹⁻₂] oder [M(O∼N∼N∼O)²⁻] sind und ein zwei- oder dreiwertiges Übergangsmetall der Gruppen 5 bis 11 enthalten. Das erfindungsgemäße Verfahren stützt sich auf in der Literatur bekannte Schiffbasen-Komplexe, wobei Komplexen des preisgünstigen 3d Metalls Kobalt eine besonders hohe Aktivität zukommt. Als besonders geeignet haben sich Komplexe vom
vom Salen-Typ, **Formel A** (R¹ = H, Me, Ph) 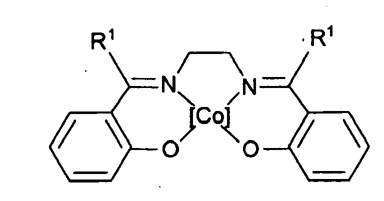 und Salophen-Typ **Formel B** (R¹ = H, Me, Ph)

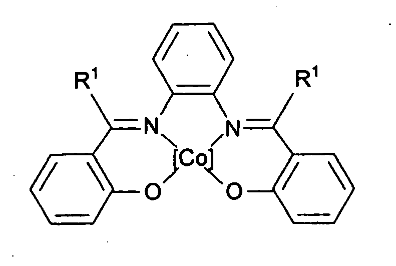

Ihr gemeinsames Strukturelement sind zwei Salicyliden-amino-Baugruppen, verknüpft durch einen Diamino-spacer X. Diese Katalysatoren haben die folgende allgemeine Leitstruktur (Formelschema I):

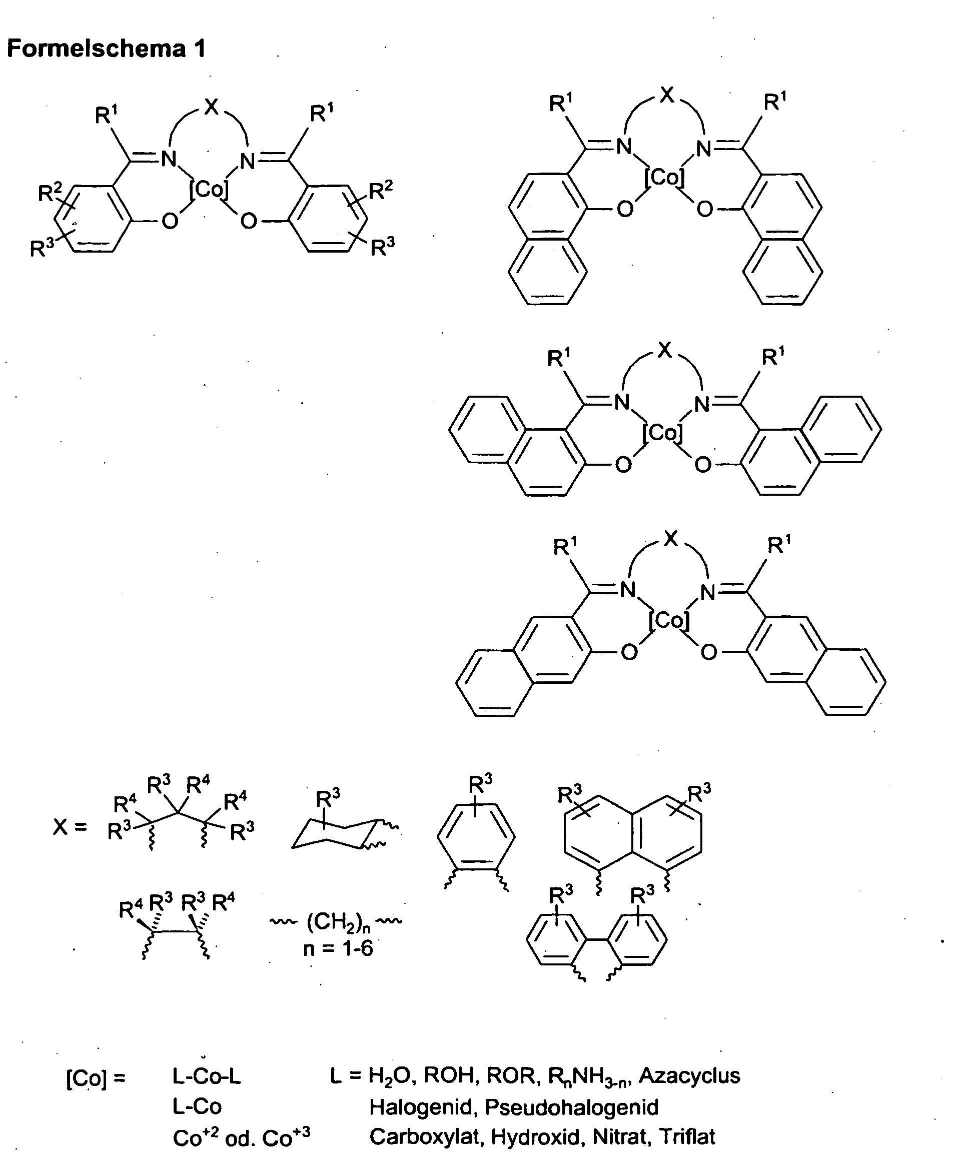

In diesen Formeln sind
R¹ = Wasserstoff, ein Alkylrest mit 1 bis 20 Kohlenstoffatomen, eine Aryl- oder Heteroarylgruppe, eine OR-Gruppe, in der R für Wasserstoff oder eine Alkylgruppe mit 1 bis 20 Kohlenstoffatomen steht, oder NRR' bedeuten, in der R und R' für Wasserstoff oder je eine Aryl- oder Alkylgruppe mit 1 bis 20 C-Atomen bedeuten oder gemeinsam ein Ringsystem mit Stickstoffatom als Heteroatom bilden können;
R² Wasserstoff, eine Alkylgruppe mit 1 bis 20 Kohlenstoffatomen, eine Arylgruppe - oder Heteroarylgruppe, die über eine oder zwei C-C-Bindungen annelliert mit dem Salicylat-Baustein verknüpft ist, eine Ketogruppe -COR oder - COOR, -COOH, Fluor, Chlor, Brom, Iod, OH, OR oder NRR', wobei R bzw. R' die vorgenannten Bedeutungen haben können und R² 1- bis 4-fach den aromatischen Ring substituieren kann;
R³ und/oder R⁴ können Wasserstoff, eine Alkylgruppe mit 1 bis 20 Kohlenstoffatomen, eine Aryl- oder Heteroarylgruppe, eine Ketogruppe -COR, -COOR, - COOH, Fluor, Chlor, Brom, Iod, OH, OR oder NRR', wobei R bzw. R' die vorgenannten Bedeutungen haben können;
L = H₂O, RₙNH₃₋ₙ, Halogenid, Sulfonat (z.B. Triflat, Tosylat), Pseudohalogenid (z.B. CN, SCN, OCN, N₃), ein Azaaromat, ein cyclisches oder acyclisches Amin bzw. Ketimin R₂C=NR', ein aliphatischer Ether oder ein aliphatischer bzw. aromatischer Alkohol sein kann. R und R' repräsentieren hierbei eine Aryl- oder Alkylgruppe mit 1 bis 20 C-Atomen, eine -O-Alkyl, NH(Alkyl) oder -N(Alkyl)₂ Gruppe. Sowohl Katalysatorkomplexe mit zweiwertigem Kobalt als auch solche mit dreiwertigem Kobalt sind katalytisch aktiv. Dieser Tatsache trägt die Formulierung [Co] Rechnung: Sind beispielsweise zwei Neutralliganden L zusätzlich zum N₂O₂²⁻ -Liganden an Kobalt [Co] koordiniert, so handelt es sich um einen neutralen Kobalt(II)- oder einen kationischen Kobalt(III)-Komplex mit nichtkoordinierendem Anion, sind ein Anion L und ein Neutralligand L' an [Co] koordiniert, so handelt es sich um einen Kobalt(III)-Neutralkomplex. Die Liganden L können in der als Substanz isolierten Katalysatorvorstufe auch fehlen. In Lösung sind L in der Regel Solvensmoleküle, sie sind in der Regel leicht durch die umgesetzten Substrate substituierbar.

X = eine beliebige Alkylen- oder Arylen-Baueinheit, die beide Imino-Stickstoffatome miteinander verknüpft.,Diese Einheit X kann über ihre zentralen Substituenten R³ bzw. R⁴ auch mit weiteren molekularen, makromolekularen oder anorganischen Bauelementen verknüpft sein.

Besonders aktiv und selektiv und daher bevorzugt sind nicht allein die hochaktiven Katalysatorkomplexe vom Salen- und Salophen-Typ, sondern weitere Katalysatoren mit [M(O∼N∼N∼O)²⁻] Liganden, die in Tabelle 1 (vgl. Beispiele) aufgeführt sind. Die Zuordnung der in Tabelle 1 verwendeten Abkürzungen zu Strukturformeln ist im folgenden Schema der besonders bevorzugten Katalysatorkomplexe angegeben:

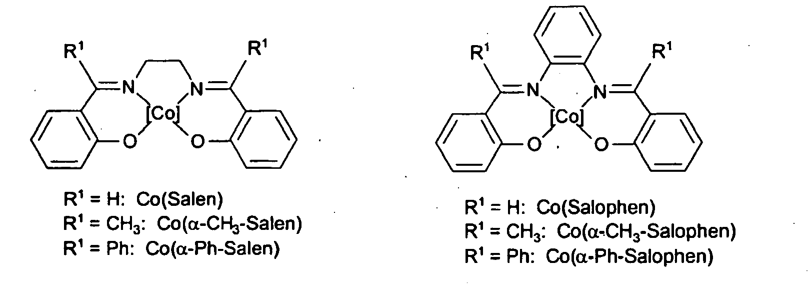

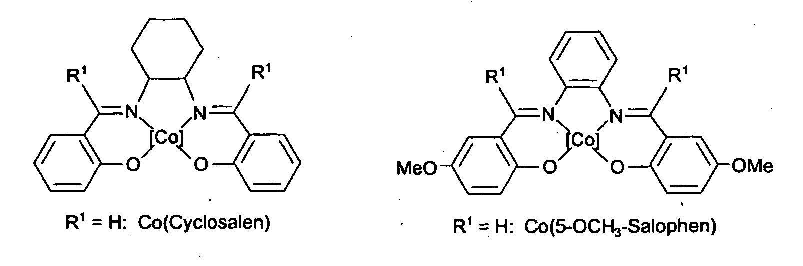

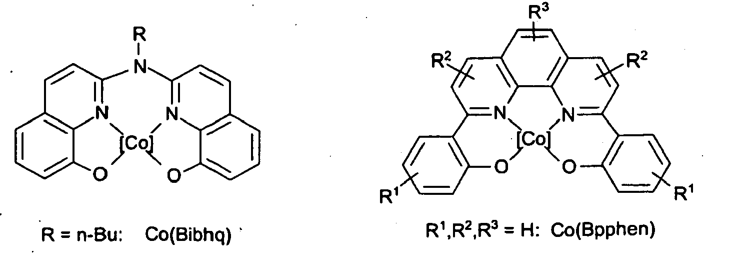

Erfindungsgemäße Katalysatoren sind folglich auch Metall-Komplexverbindungen mit anderen dianionischen N₂O₂-Chelatliganden, die Carboxylat-, Azaaromat-, Phenolat- und / oder metallierte Carbonsäureamidfunktionen in sich vereinen. Geeignete Liganden mit besonders bevorzugten Strukturelementen sind als Kobaltkomplexe im folgenden Formelschema II und Formelschema III genauer beschrieben: 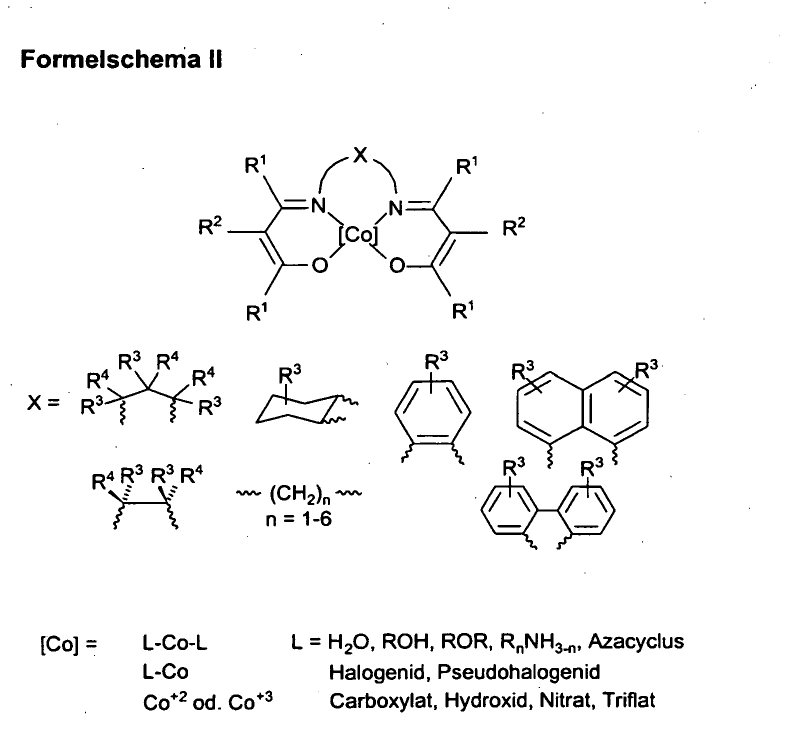 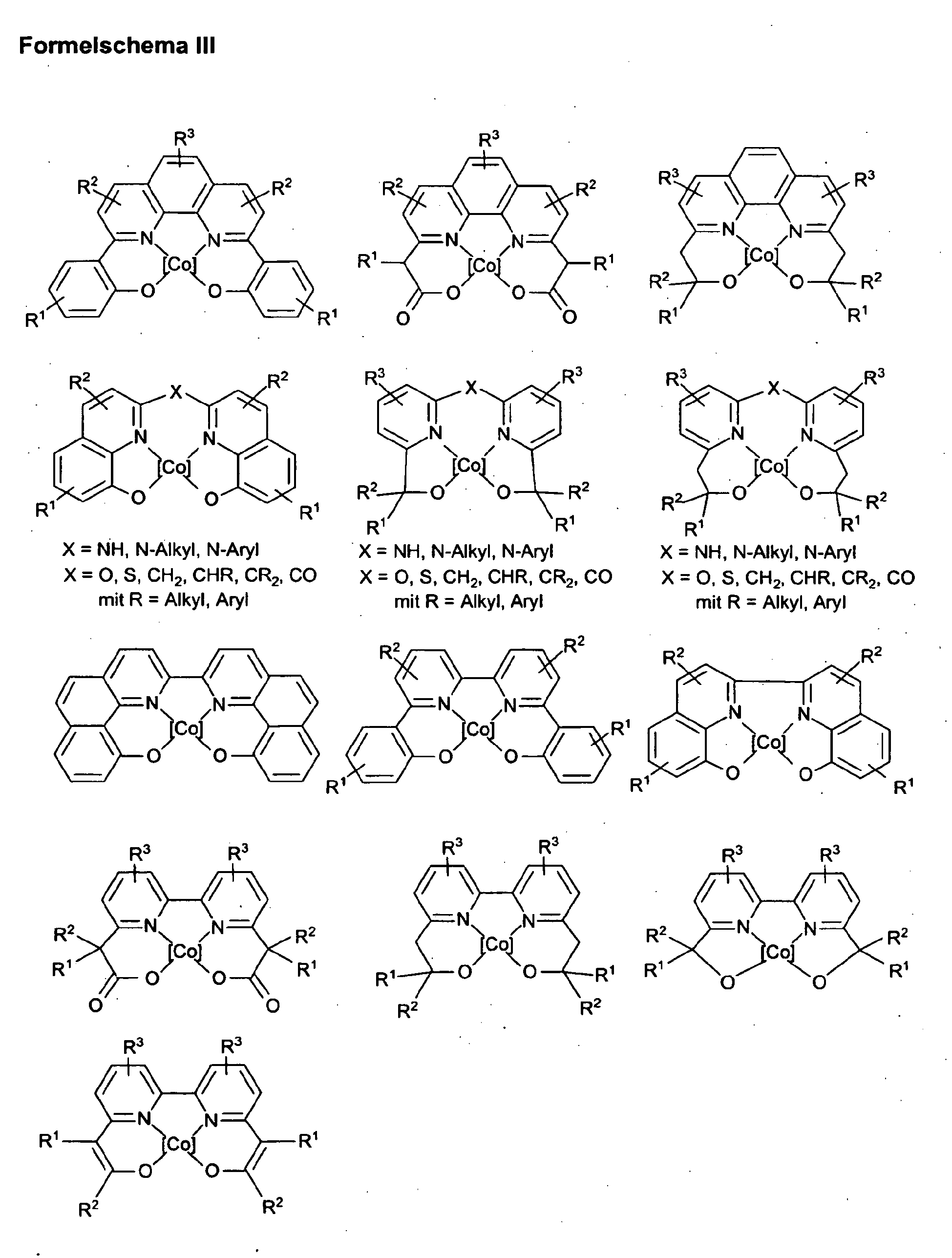 Als alternative Katalysatoren kommen statt eines dianionischen N₂O₂ Liganden auch zwei monoanionische, mindestens bidentate N,O-Chelatliganden in Frage (Formelschema IV).

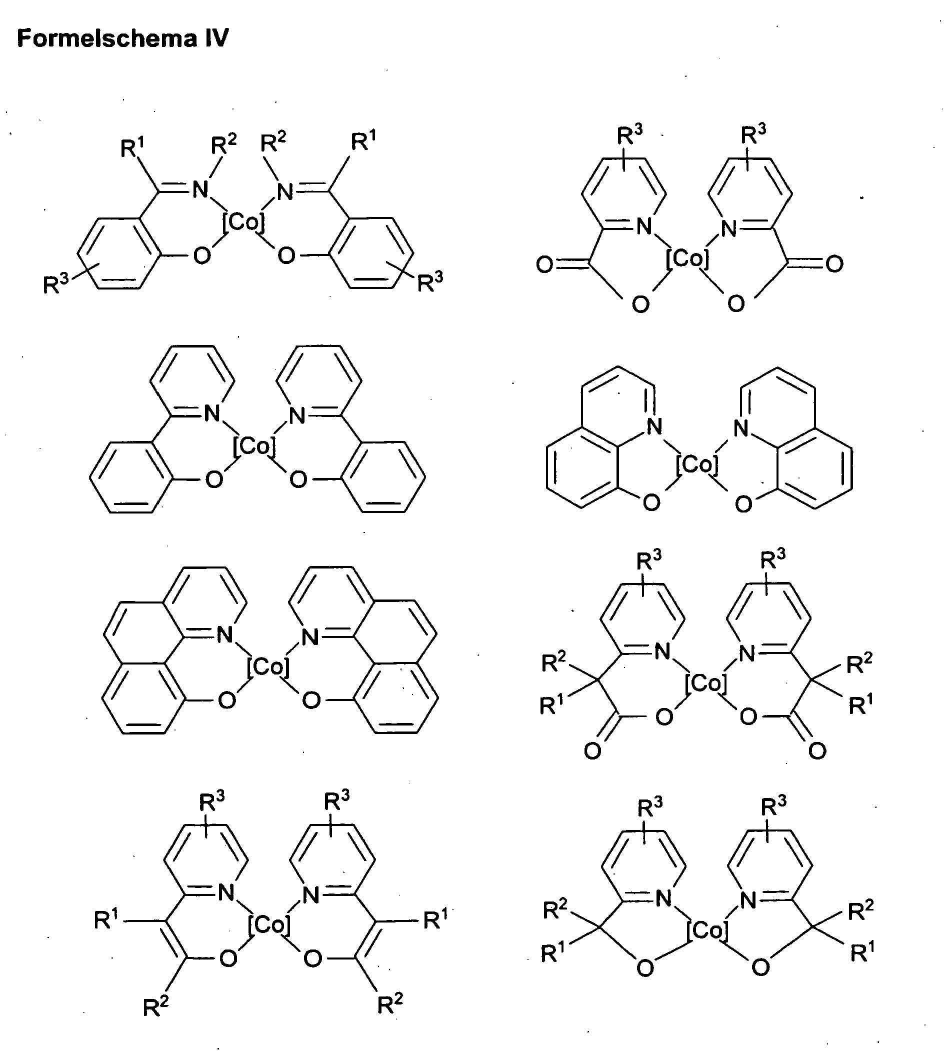

In Formelschema III und IV gilt für die Substituenten:
R¹ und / oder R² = Wasserstoff, ein Alkylrest mit 1 bis 20 Kohlenstoffatomen, eine Aryl- oder Heteroarylgruppe, eine OR-Gruppe, in der R für Wasserstoff,
eine Arylgruppe oder eine Alkylgruppe mit 1 bis 20 Kohlenstoffatomen steht, oder NRR' bedeuten, in der R und / oder R' für Wasserstoff, eine Arylgruppe oder je eine Alkylgruppe mit 1 bis 20 C-Atomen bedeuten oder gemeinsam ein Ringsystem mit Stickstoffatom als Heteroatom bilden können;
R³ Wasserstoff, eine Alkylgruppe mit 1 bis 20 Kohlenstoffatomen, eine Arylgruppe - oder Heteroarylgruppe, die über eine oder zwei C-C-Bindungen annelliert mit dem Salicylat-Baustein verknüpft ist, eine Ketogruppe -COR, COOR, COOH, Fluor, Chlor, Brom, Iod, OH, OR oder NRR', die die vorgenannten Bedeutungen haben können, wobei R³ 1- bis 4-fach den aromatischen Ring substituieren kann;

Mit diesen Katalysatoren lassen sich Umsätze und Selektivitäten von über 95%, d.h. Ausbeuten von über 90%, bezogen auf den eingesetzten Nitroaromaten oder bezogen auf ein Gemisch aus Nitroaromat und Anilin, erzielen.

Ar-NO₂ + 3 CO + ROH - Cat. → Ar-NH-CO-OR + 2 CO₂

Die Umsetzung erfolgt in gleicher Weise auch mit Di- und Polynitroverbindungen. Es lässt sich auch das Methylcarbamat des TDI gewinnen, wie Beispiel 5 offenlegt.

Das erfindungsgemäße Verfahren lässt sich mit besonders guten Ausbeuten und Erträgen durchführen, wenn das durch Reduktion der aromatischen Nitroverbindung entstehende Anilin in Mengen von bis zu 200 Mol-% eingesetzt wird. Durch Zusatz eines Anilins lassen sich erheblich bessere Ausbeuten erzielen, wenn das Verhältnis von Nitroaromat zu Anilin 100 : 10 Mol-% beträgt (Beispiele 1 und 2 sowie Tabelle 2). Die besten Ausbeuten wurden erzielt bei einem Verhältnis von Nitroaromat zu Anilin von 100 : 200 Mol-% (Beispiel 3). Die folgende Reaktionsgleichung beschreibt diesen vorteilhaften Grenzfall der umgesetzten Stoffe des erfindungsgemäßen Verfahrens:

ArNO₂ + 2 Ar-NH₂ + 3 CO + 3 ROH - Cat. → 3 Ar-NH-CO-OR + 2 H₂O

Die Umsetzung erfolgt in gleicher Weise auch mit Di- und Polynitroverbindungen. Bei den Varianten, in denen der Nitroaromat in Gegenwart von 1-2 Moläquivalenten Anilin reduktiv carbonyliert wird, hat es sich als vorteilhaft erwiesen, das bei der Reaktion gebildete Wasser durch chemische oder physikalische Methoden zu einem gewissen Anteil aus dem Reaktionsgemisch zu entfernen, denn Wasser kann unter ungünstigen Bedingungen die Hydrolyse des gebildeten Carbamats bewirken. Dennoch werden auch ohne die Entfernung von Wasser meist Selektivitäten größer als 90 Prozent problemlos erreicht (vergleiche Beispiel 5). Spuren an Wasser sind andererseits förderlich, denn Wasser und CO sind an der Reduktion von Nitroaromaten zu intermediär unter bestimmten Bedingungen auftretenden Anilinderivaten beteiligt.

Besonders bevorzugt sind die erfindungsgemäßen Komplexverbindungen mit Kobalt als Zentralatom. Jedoch werden auch mit Mangan, Chrom, Nickel, Kupfer sowie einigen wenigen 4d-Metallen, wie Rhodium und Ruthenium und 5d-Metallen, wie Osmium und Platin gute Ergebnisse erzielt. Der Metallkomplexkatalysator wird in einer Konzentration von 0.05 bis 10 Mol-%, vorzugsweise 0.1 bis 3 Mol%, eingesetzt.

Die Umsetzung kann gemäß einer bevorzugten Ausgestaltung in einem Autoklaven bei Temperaturen von etwa 80 - 260°C unter einem Kohlenmonoxiddruck von etwa 10 - 100 bar in einem aliphatischen oder aromatischen Alkohol durchgeführt werden.

Vorzugsweise wird die Reaktion in einem Autoklaven bei Temperaturen von 80 bis 220°C unter einem Kohlenmonoxiddruck von 10 bis 100 bar in Gegenwart eines aliphatischen oder aromatischen Alkohols durchgeführt. Besonders bewährt hat sich Methanol.

Besonders gute Ergebnisse werden in der in Gegenwart eines sauren Promotors erzielt. Dieser wird in unterschiedlichen Konzentrationen, vorzugsweise jedoch im äquimolaren Verhältnis relativ zum Katalysatorkomplex eingesetzt (vgl. Tabelle 3 in den Beispielen). Der saure Promotor ist entweder eine LewisSäure, etwa BF₃ oder das Tosylat B(OTs)₃, die in Verbindung mit Wasser oder der OH-enthaltenden organischen Komponente dieses Verfahrens eine Protonensäure liefert, oder der saure Promotor ist selbst eine Protonensäure. Besonders bevorzugt sind starke Protonensäuren mit einem pKs Wert kleiner als 5. Hierfür haben sich besonders Sulfonsäuren wie die p-Toluolsulfonsäure, Trifluormethyl- oder Nonafluor-n-butylsulfonsäure, Camphersulfonsäure, aber auch SO₃H-Gruppe tragende Polymersäuren, beispielsweise saure lonenaustauscherharze auf Basis von sulfonierten Polystyrolen, aber auch anorganische Festkörper als Säuren wie etwa saure Alumosilikate (Zeolithe) bewährt. Andere Säure-Promotoren umfassen aliphatische und aromatische, molekulare und polymergebundene Carbonsäuren, beispeilsweise Trifluoressigsäure und höhere Homologe, p-Chlorbenzoesäure, Dicarbonsäuren, Schwefelsäurederivate, beispielsweise Fluorsulfonsäure, Phosphorsäure und ihre partiell veresterten aromatischen und aliphatischen Derivate, z.B. (RO)₂PO(OH), aromatische und aliphatische Phosphonsäuren R-PO(OH)2 und Phosphinsäuren R₂PO(OH). Schließlich finden rein anorganische Mineralsäuren, Tetrafluorborsäure und Hexafluorphorphorsäure als Promotoren Verwendung.

Bei dem erfindungsgemäßen Verfahren können sich durch die reduktive Carbonylierung als Zwischenprodukte zunächst Anilinderivate bilden, weiterhin bei bewusst zugesetztem Anilin auch N,N'-Diarylharnstoffe (vgl. Beispiel 6) und N-Arylcarbamate (Urethane) bilden. Aus letzteren kann der Alkohol durch thermische Behandlung in einfacher Weise abgespalten werden. Somit ergibt sich folgende besonders vorteilhafte autokatalytische Reaktionssequenz, die durch Katalysatoren der hier beschriebenen Art in Gegenwart der Säure-Promotoren ermöglicht wird:

Ar-NO₂ + 3 CO + H₂O - Cat. → Ar-NH₂ + 3 CO₂

ArNO₂ + 2 Ar-NH₂ + 3 CO + 3 ROH - Cat. → 3 Ar-NH-CO-OR + 2 H₂O

Ar-NH-CO-OR - Δ → Ar-NCO + ROH

In gleicher Weise werden auch Di- und Polynitroverbindungen umgesetzt (vgl. Beispiel 5).

Die thermolytische Spaltung isolierter Carbamate zu Isocyanaten und organischer Hydroxyverbindung ist eine bekannte Technik. Es ist aber auch möglich, die hier beschriebenen Reaktionen so durchzuführen, dass Anilinderivate und N-Arylcarbamate nur in Spuren gebildet und Arylisocyanate direkt unter den Reaktionsbedingungen bevorzugt als Endprodukt gebildet werden. Zu diesem Zweck werden die Reaktionen entweder homogen, vorzugsweise nicht in Alkoholen als Lösemittel, sondern in einem inerten Lösemittel, beispielsweise Chlorbenzol oder Toluol in Gegenwart geringer Mengen an Alkohol, durchgeführt. Auch hat sich zu diesem Zweck eine heterogene Trägerung der Katalysatorkomplexe und Promotoren auf anorganischen oder organischen Trägermaterialien, beispielsweise auf saurem Aluminiumoxid Al₂O₃, auf Silicagel, auf sulfoniertem Polystyrol, in alumosilikatischen Käfigstrukturen wie Zeolith Y oder mesoporösen Silica-Materialien wie MCM-41 oder MCM-48 bewährt (zum Begriff MCM: Zhao et al, Ind. Eng. Chem. Res. 35 (1996) 2075).

Ausgangsstoff für das erfindungsgemäße Verfahren können sowohl Mononitroals auch Dinitro-Verbindungen sein. Beim Einsatz von Dinitro-Verbindungen entstehen zunächst Mono-, dann Di-N-Arylcarbamate, aus denen durch Thermolyse Di-N-Arylisocyanate gebildet werden können. Es ist aber auch möglich, die Reaktion so zu leiten, dass direkt Di-N-Arylisocyanate entstehen.

Das erfindungsgemäße Verfahren wird durch die nachfolgenden Beispiele näher erläutert:

### Beispiel 1

### Umsetzung:

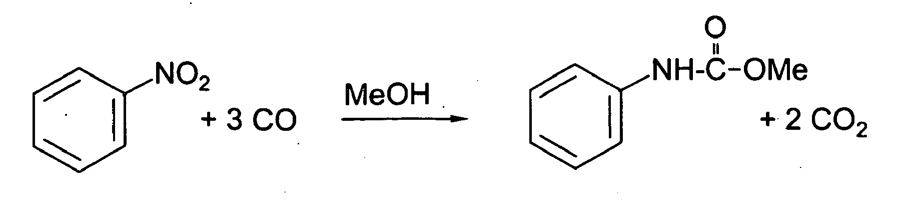

Die vorstehend genannte Reaktion wurde in einem 100 cm³ Edelstahlautoklaven mit Polytetrafluorethylenbecher als Insert durchgeführt. In dem Autoklaven wurden 0,196 g (0,5 mmol) N,N'-Bis(salicyliden)-1,2-phenylen-diaminocobalt (II) monohydrat, 0,0870 g (0,5 mmol) p-Toluolsulfonsäure als Promotor oder Cokatalysator, 0,0548 g (0,59 mmol) Anilin, 0,6534 g (5,3 mmol) Nitrobenzol und 4,172 g (13,05 mmol) Methanol gemischt. Der Autoklav wurde dreimal mit Stickstoffgas gespült und dann bei Raumtemperatur Kohlenmonoxidgas unter einem Druck von 50 bar eingeleitet.

Nachdem die Gaszufuhr des Autoklaven unterbrochen worden war, wurde der Autoklav in einen Aluminiumheizblock eingesetzt, der auf 200°C erhitzt war. Innerhalb von 5 Minuten erreichte der Autoklav eine Reaktionstemperatur von 170°C, die dann konstant gehalten wurde. Nach einer Reaktionsdauer von 7 Stunden wurde der Autoklav durch kaltes Wasser und Eis abgekühlt, bis er nach etwa 5 Minuten Raumtemperatur erreichte. Die Reaktionsmischung wurde dann qualitativ und quantitativ gaschromatographisch untersucht, wobei Naphthalin als interner Standard diente. Die Umwandlung von Nitrobenzol betrug 100%, die Ausbeute an Methyl-N-phenylcarbamat 99%.

### Beispiel 2

### Umsetzung

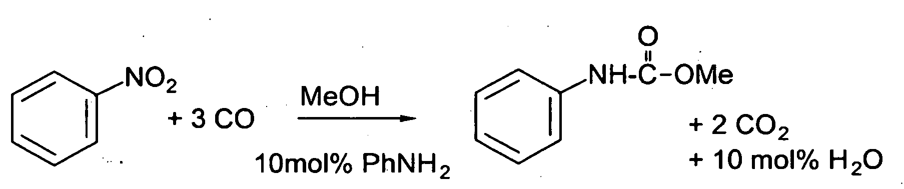

Die im Beispiel 1 genannte Reaktion wurde erneut in einem. Edelstahlautoklaven mit Polytetrafluorethylenbecher von 100 cm³ Fassungsvermögen durchgeführt. 0,324 g (1,0 mmol) N,N'-Bis(salicyliden)-ethylendiaminocobalt (II), 0,172 g (=1,0 mmol) p-Toluolsulfonsäure, 0,093 g (1,0 mmol) Anilin, 1,251 g (10,2 mmol) Nitrobenzol, 8,042 g (25,1 mmol) Methanol wurden in dem Autoklaven gemischt. Der Autoklav wurde dreimal mit Stickstoffgas gespült und dann bei Raumtemperatur Kohlenmonoxid unter einem Druck von 50 bar eingeleitet.

Nachdem die Gaszufuhr des Autoklaven unterbrochen worden war, wurde er in einen Aluminiumheizblock eingesetzt, der auf 200°C vorgeheizt war. Nach 5 Minuten erreichte der Autoklav eine Reaktionstemperatur von 170°C, die dann konstant gehalten wurde. Nach einer Reaktionsdauer von 7 Stunden wurde der Autoklav mit kaltem Wasser und Eis gekühlt und erreichte nach 5 Minuten Raumtemperatur. Die Reaktionsmischung wurde dann qualitativ und quantitativ durch Gaschromatographie untersucht, wobei Naphthalin als interner Standard diente. Die Umwandlung von Nitrobenzol betrug 100%, die Ausbeute von Methyl-N-phenylcarbamat 99,0% und die Selektivität von Methyl-N-phenylcarbamat betrug ebenfalls 99%.

### Beispiel 3

### Umsetzung:

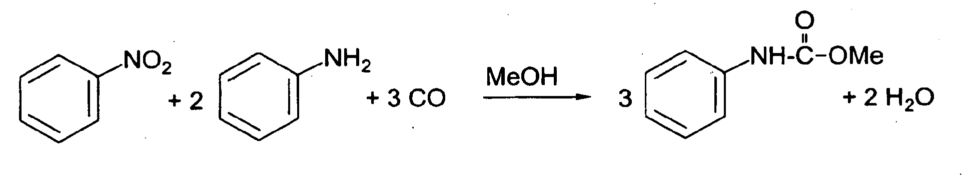

Die vorstehend genannte Reaktion wurde in einem mit einem Edelstahlautoklaven mit Polytetrafluorethylenbecher von 100 cm³ Fassungsvermögen als Insert durchgeführt. 0,0866 g (0,22 mmol) N,N'-Bis(salicyliden)-1,2-phenylendiaminocobalt(II) monohydrat, 0,0341 g (0,2 mmol) p-Toluolsulfonsäure, 1,8650 g (20,05 mmol) Anilin, 1,2354 g (10,0 mmol) Nitrobenzol und 8,1370 g (254,3 mmol) Methanol wurden in dem Autoklaven gemischt. Der Autoklav wurde dann fünfmal mit Stickstoffgas gespült und bei Raumtemperatur mit Kohlenmonoxidgas unter einem Druck von 50 bar gefüllt.

Nach Unterbrechung der Gaszufuhr wurde der Autoklav in einen Aluminiumheizblock eingesetzt, der auf 200°C vorgeheizt war. Innerhalb von 5 Minuten erreichte der Autoklav eine Reaktionstemperatur von 170°C, die dann konstant gehalten wurde. Nach einer Reaktionsdauer von 7 Stunden wurde der Autoklav mit kaltem Wasser und Eis gekühlt und erreichte Raumtemperatur nach etwa 5 Minuten. Die Reaktionsmischung wurde qualitativ und quantitativ gaschromatographisch untersucht, wobei Naphthalin als interner Standard diente. Die Umwandlung von Nitrobenzol betrug 100%, die Ausbeute von Methyl-N-phenylcarbamat 97,6%, während die Selektivität von Methyl-N-phenylcarbamat ebenfalls 97,6% betrug.

### Beispiel 4 (Vergleichsbeispiel)

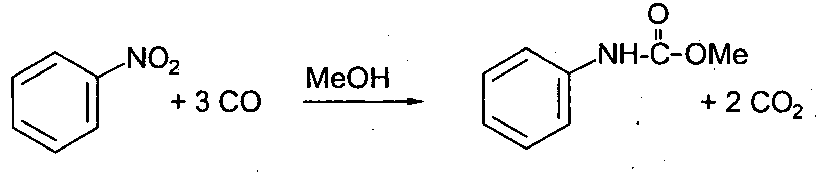

Die vorstehend genannte Reaktion wurde in einem 100 cm³ Edelstahlautoklaven mit Polytetrafluorethylenbecher als Insert durchgeführt. 0.0993 g (0,25 mmol) N,N'-Bis(salicyliden)-1,2-phenylendiaminocobalt(II)-monohydrat, 0,0440 g (0,26mmol) p-Toluolsulfonsäure, 0,6340 g (5,15 mmol) Nitrobenzol, 4,3840 g (13,7 mmol) Methanol wurden in dem Autoklaven gemischt. Es wurde kein Anilin zugegeben. Der Autoklav wurde fünfmal mit Stickstoff gespült und dann bei Raumtemperatur Kohlenmonoxidgas unter einen Druck von 50 bar eingeleitet.

Nachdem die Gaszufuhr des Autoklaven unterbrochen worden war, wurde er in einen Aluminiumheizblock eingesetzt, der auf 200°C vorgeheizt war. Innerhalb von 5 Minuten erreichte der Autoklav eine Reaktionstemperatur von 170°C, die dann konstant gehalten wurde. Nach einer Reaktionsdauer von 7 Stunden wurde der Autoklav durch kaltes Wasser und Eis gekühlt und erreichte nach 5 Minuten Raumtemperatur. Die Reaktionsmischung wurde dann qualitativ und quantitativ gaschromatographisch untersucht, wobei Naphthalin als interner Standard diente. Die Umwandlung von Nitrobenzol betrug 48,3%, die Ausbeute an Methyl-N-phenyl-carbamat betrug 41,8%, die Selektivität von Methyl-N-phenyl-carbamat 86,5%.

Weitere Beispiele zur Aktivität ausgewählter Katalysatorkomplexe, zum Effekt des zugefügten Anilins und zum Einfluss des Säurepromotors sind in Tabelle 1-3 zusammengefasst.

### Tabelle 1: Beispiele verwendeter Katalysatorkomplexe in der reduktiven Carbonylierung

einheitliche Bedingungen, einzige Variable ist der Katalysator
n_{Kat}/n_{PhNO2} = 1/200
n_{Kat}/n_{Promotor} = 1/1
n_{PhNH2}/n_{PhNO2} = 2/1
n_{MeOH}/n_{PnNO2} = 25/1
Promotor = p-TsOH
Temperatur = 170°C
Reaktionsdauer = 7 h
CO-Druck = 50 bar
Die Durchführung erfolgt, abgesehen von eingewogenen Mengen wie in Patentbeispiel 3 beschrieben. Die hier verwendeten Abkürzungen sind im Textteil erläutert.

| **Eintrag** | **Katalysator** | **Umsatz von PhNO₂+PhNH₂ [%]** | **PhNHCOOCH₃ Ausbeute (bezogen auf PhNO₂+PhNH₂)** [%] | **PhNHCOOCH₃ Selektivität (bezogen auf PhNO₂+PhNH₂)** [%] |
|---|---|---|---|---|
| **1** | [CoSalophen·H₂O] | 49.7 | 51.7 | 100 |
| **2** | [CoSalophen] | 42.1 | 46.9 | 100 |
| **3** | [CoSalen] | 33.6 | 30.7 | 91.4 |
| **4** | [Co-α-CH₃-Salen] | 61.5 | 51.2 | 83.3 |
| **5** | [Co-α-Ph-Salen] | 54.0 | 42.7 | 79.1 |
| **6** | [CoCyclosalen] | 46.0 | 42.5 | 92.4 |
| **7** | [Co-5-OCH₃-salophen] | 39.4 | 31.4 | 79.7 |
| **8** | [CoBpphen]^{[a]} | 100 | 66.0 | 66.0 |
| **9** | [CoBibhg]^{a} | 78.0 | 66.0 | 84.0 |

| | | | | |
|---|---|---|---|---|
| ^{[a]} n_{Kat}/n_{PhNO2} = 1/50 | | | | |

### Tabelle 2: Effekt von zugesetztem Anilin auf die reduktive Carbonylierung

einheitliche Reaktionsbedingungen, einzige Variable ist das Anilin zu Nitrobenzol Verhältnis
Katalysator: [Co"Salophen]
n_{Kat}/n_{PhNO2} = 1/200
n_{Kat}/n_{Promotor} = 1/1
n_{MeOH}/n_{PhNO2} = 25/1
Promotor = p-TsOH
Temperatur = 170°C
Reaktionsdauer = 7 h
CO-Druck = 50 bar

| **Eintrag** | **n_{PhNH2}/n_{PhNO2}** | **Umsatz von PhNO₂+PhNH₂ [%]** | **PhNHCOOCH₃ Ausbeute (bezogen auf PhNO₂+PhNH₂) [%]** | **PhNHCOOCH₃ Selektivität (bezogen auf PhNO₂+PhNH₂) [%]** |
|---|---|---|---|---|
| **1** | 0 | 5.0 | 1.8 | 36.0 |
| **2** | 0.5 | 32.8 | 33.3 | 100 |
| **3** | 1 | 49.3 | 49.0 | 99.4 |
| **4** | 1.5 | 56.6 | 47.9 | 84.6 |
| **5** | 2 | 54.4 | 51.5 | 94.7 |
| **6** | 3 | 57.5 | 51.9 | 90.3 |
| **7** | 5 | 42.7 | 33.6 | 78.7 |

### Tabelle 3: Beispiele von Säurepromotoren in der reduktiven Carbonylierung

Reaktionsbedingungen einheitlich, Promotor äquimolar zu Katalysator einzige Variable
Katalysator: [Co"Salophen]
T_{Kat}/n_{PhNO2} = 1/200
n_{Kat}/n_{Promotor} = 1/1
n_{PhNH2}/n_{PhNO2} = 2/1
n_{MeOH}/n_{PhNO2} = 25/1
Temperatur = 170°C
Reaktionsdauer = 7 h
CO-Druck = 50 bar

| **Eintrag** | **Promotor** | **Umsatz von PhNO₂+PhNH₂ [%]** | **PhNHCOOCH₃ Ausbeute (bezogen auf PhNO₂+PhNH₂) [%]** | **PhNHCOOCH₃ Selektivität (bezogen auf PhNO₂+PhNH₂) [%]** |
|---|---|---|---|---|
| **1** | ohne Promotor | 15.5 | 8.7 | 56.1 |
| **2** | p-CH₃-C₆H₅-SO₃H | 47.5 | 46.9 | 98.7 |
| **3** | C₄F₉SO₃H | 77.3 | 74.1 | 95.9 |
| **4** | CF₃COOH | 56.3 | 51.2 | 90.9 |
| **5** | CF₃SO₃H | 81.6 | 80.1 | 98.2 |
| **6** | Amberlite IR-120 (feucht) 1:1 | 30.8 | 21.8 | 70.8 |
| **7** | Amberlite (IR-120 (wasserfrei) 1:1 | 25.6 | 23.1 | 90.2 |

### Beispiel 5

### Vorschrift zur Isolierung des Bis(methylcarbamats) des Toluol-2,4-diisocyanats (TDI):

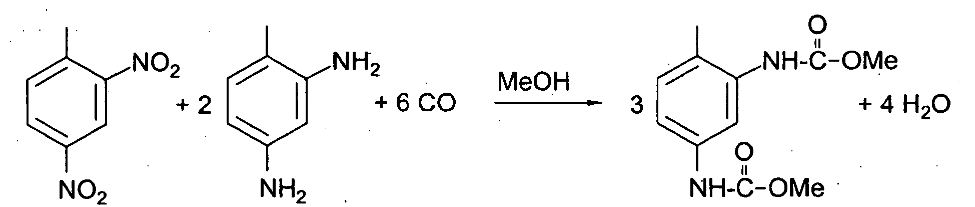

Die Reaktion wurde in einem Edelstahlautoklaven mit Polytetrafluorethylenbecher (100 cm³ Fassungsvermögen) durchgeführt. Es wurden 0,0186 g (0,05 mmol) N,N'-bis(salicyliden)-1,2-phenylendiaminocobalt(II), 0,0086g (0,05 mmol) p-Toluolsulfonsäure, 0,6103 (5,0 mmol) 2,4-Diaminoanilin, 0,4550g (2,5 mmol) 2,4-Dinitrotoluol, 5,07 g (15,8 mmol) Methanol in dem Autoklaven gemischt. Der Autoklav wurde dreimal mit Stickstoffgas gespült, dann wurden 50 bar Kohlenmonoxid aufgedrückt. Nachdem Unterbrechung der Gaszufuhr wurde der Autoklav in einen Aluminiumheizblock eingesetzt, der auf 200°C vorgeheizt war. Nach 5 Minuten erreichte der Autoklav eine Reaktionstemperatur von 170°C, die dann konstant gehalten wurde. Nach einer Reaktionsdauer von 6 Stunden wurde der Autoklav mit kaltem Wasser und Eis gekühlt und erreichte nach 5 Minuten Raumtemperatur. Die Reaktionsmischung wurde abfiltriert und der erhaltene Feststoff drei Mal mit kaltem Methanol gewaschen. Anschließend wurde der Feststoff 5 Stunden lang im Hochvakuum getrocknet. Die Charakterisierung erfolgte per ¹H-NMR, MS, CHN-Analyse und Schmelzpunkt. Es wurden 1,6520 g TDI-Methylcarbamat isoliert, was einer Ausbeute von 92,6 % entspricht.

### Beispiel 6

### Vorschrift zur Isolierung von Harnstoffen bei kürzerer Reaktionszeit und höherem Anilin-Anteil:

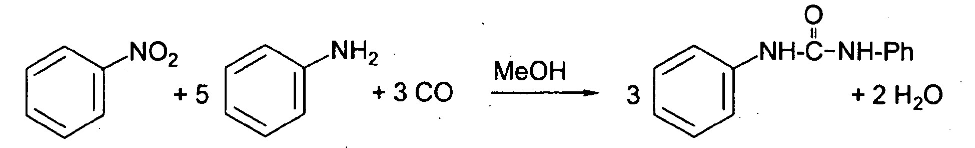

Die obenstehende Reaktion wurde in einem Edelstahlautoklaven mit Polytetrafluorethylenbecher (100 cm³ Fassungsvermögen) durchgeführt. Es wurden 0.0373 g (0,1 mmol) N,N'-bis(salicyliden)-1,2-phenylendiaminocobalt(II), 0,0172 g (0,1 mmol) p-Toluolsulfonsäure, 4,6498 g (50,0 mmol) Anilin, 1.2318 g (10,0 mmol) Nitrobenzol und 8,69 g (10 mmol) Toluol in dem Autoklaven gemischt. Der Autoklav wurde dreimal mit Stickstoffgas gespült, dann wurden 50 bar Kohlenmonoxid aufgedrückt. Nachdem Unterbrechung der Gaszufuhr wurde der Autoklav in einen Aluminiumheizblock eingesetzt, der auf 200°C vorgeheizt war. Nach 5 Minuten erreichte der Autoklav eine Reaktionstemperatur von 170°C, die dann konstant gehalten wurde. Nach einer Reaktionsdauer von 6 Stunden wurde der Autoklav mit kaltem Wasser und Eis gekühlt und erreichte nach 5 Minuten Raumtemperatur. Die Reaktionsmischung wurde abfiltriert und der erhaltene Feststoff mit Toluol (3 x 10 ml) gewaschen. Anschließend wurde der Feststoff 4 Stunden lang im Hochvakuum getrocknet. Die Charakterisierung erfolgte per ¹H-NMR, MS, CHN-Analyse und Schmelzpunkt. Es wurden in diesem noch nicht optimierten Versuch 3,0414 g N,N'-Diphenylharnstoff isoliert, was einer Ausbeute von 23,9 % entspricht.

## Patentansprüche

1. Verfahren zur Herstellung von N-Arylcarbamaten (Urethanen) und/oder N-Arylisocyanaten durch reduktive Carbonylierung von aromatischen Nitroverbindungen in Gegenwart von Kohlenmonoxid und organischen, Hydroxylgruppen tragenden Verbindungen, **dadurch gekennzeichnet, dass** die Carbonylierung
- in Anwesenheit eines Kobalt-Komplexkatalysators, der anionische N,O-Chelatliganden des allgemeinen Typs [M(N∼O)¹⁻₂] oder [M(O∼N∼N∼O)²] enthält und
- in Gegenwart von bis zu 2 Moläquivalenten eines aromatischen Amins im Verhältnis zur eingesetzten aromatischen Nitroverbindung
durchgeführt wird.

2. Verfahren nach den Anspruch 1, **dadurch gekennzeichnet, dass** der Kobalt-Komplexkatalysator in einer Konzentration von 0.1 - 10 mol% eingesetzt wird.

3. Verfahren nach den Ansprüchen 1 und 2, **dadurch gekennzeichnet, dass** die Umsetzung in einem Autoklaven bei Temperaturen von 80 - 220°C unter einem Kohlenmonoxiddruck von 10 - 100 bar in Gegenwart eines aliphatischen oder aromatischen Alkohols durchgeführt wird.

4. Verfahren nach Anspruch 3, **dadurch gekennzeichnet, dass** die Umsetzung in Methanol durchgeführt wird.

5. Verfahren nach den Ansprüchen 1 bis 4, **dadurch gekennzeichnet, dass** die Umsetzung in Gegenwart eines sauren Promotors durchgeführt wird, der einen pKₛ-Wert unter 5 aufweist.

6. Verfahren nach den Ansprüchen 1 bis 5, **dadurch gekennzeichnet, dass** die Umsetzung in Gegenwart von p-Toluolsufonsäure oder einer SO₃H-Gruppen tragenden, polymeren Säure durchgeführt wird.

7. Verfahren nach den Ansprüchen 1 bis 6, **dadurch gekennzeichnet, dass** die N-Arylisocyanate aus den zunächst entstehenden N-Arylcarbamaten (Urethanen) durch Abspaltung von Alkohol gebildet werden.

8. Verfahren nach den Ansprüchen 1 bis 7, **dadurch gekennzeichnet, dass** die Umsetzung in einem einstufigen Verfahren direkt zu N-Arylisocyanaten führt, wobei die Reaktionen mit einer heterogenen Trägerung der Katalysatorkomplexe und Promotoren auf anorganischen oder organischen Trägermaterialien durchgeführt werden.

9. Verfahren nach den Ansprüchen 1 bis 8, **dadurch gekennzeichnet, dass** aromatische Dinitroverbindungen in die entsprechenden Di-N-Arylcarbamate und/oder Di-N-Arylisocyanate umgesetzt werden

## Claims

1. A method for the production of N-aryl carbamates (urethanes) and/or N-aryl isocyanates by reductive carbonylation of aromatic nitro compounds in the presence of carbon monoxide and organic compounds bearing hydroxyl groups, **characterised in that** the carbonylation is carried out
- in the presence of a cobalt complex catalyst which contains anionic N,O-chelating ligands of the general type [M(N∼O)¹⁻₂] or (M(O∼N∼N∼O)²⁻], and
- in the presence of up to 2 molar equivalents of an aromatic amine in relation to the aromatic nitro compound used.

2. The method according to claim 1, **characterised in that** the cobalt complex catalyst is used in a concentration of 0.1-10 mol %.

3. The method according to claims 1 and 2, **characterised in that** the reaction is carried out in an autoclave at temperatures of 80-220 °C at a carbon monoxide pressure of 10-100 bar in the presence of an aliphatic or aromatic alcohol.

4. The method according to claim 3, **characterised in that** the reaction is carried out in methanol.

5. The method according to claims 1 to 4, **characterised in that** the reaction is carried out in the presence of an acid promoter which has a pKₛ value below 5.

6. The method according to claims 1 to 5, **characterised in that** the reaction is carried out in the presence of p-toluene sulphonic acid or a polymer acid bearing SO₃H groups.

7. The method according to claims 1 to 6, **characterised in that** the n-aryl isocyanates are formed from the initially produced N-aryl carbamates (urethanes) by cleaving of alcohol.

8. The method according to claims 1 to 7, **characterised in that** the reaction leads directly to N-aryl isocyanates in a single-stage process, the reactions being carried out with a heterogeneous support of the catalyst complexes and promoters on inorganic or organic supporting materials.

9. The method according to claims 1 to 8, **characterised in that** aromatic dinitro compounds are converted into the corresponding Di-N-aryl carbamates and/or Di-N-aryl isocyanates.

## Revendications

1. Procédé de préparation d'N-arylcarbamates (uréthanes) et/ou d'N-arylisocyanates par carbonylation réductrice de composés nitro aromatiques en présence de monoxyde de carbone et de composés organiques porteurs de groupes hydroxyle, **caractérisé en ce que** ladite carbonylation est réalisée
- en présence d'un catalyseur au complexe de cobalt contenant des ligands chélatants N,O de type général [M(N∼O)¹⁻₂] ou [M(O∼N∼N∼O)²⁻] et
- en présence de jusqu'à 2 équivalents molaires d'une amine aromatique, par rapport au composé nitro aromatique mis en oeuvre.

2. Procédé selon la revendication 1, **caractérisé en ce que** ledit catalyseur au complexe de cobalt est mis en oeuvre dans une concentration comprise entre 0,1 et 10 % en moles.

3. Procédé selon les revendications 1 et 2, **caractérisé en ce que** ladite réaction est réalisée dans un autoclave à des températures comprises entre 80 et 220 °C, sous une pression de monoxyde de carbone comprise entre 10 et 100 bar, en présence d'un alcool aliphatique ou aromatique.

4. Procédé selon la revendication 3, **caractérisé en ce que** ladite réaction est réalisée dans du méthanol.

5. Procédé selon les revendications 1 à 4, **caractérisé en ce que** ladite réaction est réalisée en présence d'un promoteur acide présentant un pKₛ inférieur à 5.

6. Procédé selon les revendications 1 à 5, **caractérisé en ce que** ladite réaction est réalisée en présence d'acide paratoluènesulfonique ou d'un acide polymère porteur de groupes SO₃H.

7. Procédé selon les revendications 1 à 6, **caractérisé en ce que** lesdits N-arylisocyanates sont formés, par élimination d'alcool, à partir desdits N-arylcarbamates (uréthanes) obtenus dans un premier temps.

8. Procédé selon les revendications 1 à 7, **caractérisé en ce que** ladite réaction permet d'obtenir lesdites N-arylisocyanates directement dans un procédé à étape unique, les réactions étant réalisées avec une mise sur support hétérogène des complexes catalyseurs et des promoteurs sur des matériaux de support inorganiques ou organiques.

9. Procédé selon les revendications 1 à 8, **caractérisé en ce que** l'on fait réagir des composés dinitro aromatiques pour obtenir les di-N-arylcarbamates et/ou les di-N-arylisocyanates correspondants.
